# EUROPÄISCHE PATENTSCHRIFT

(11) **EP 3 171 776 B1**
(45) Veröffentlichungstag und Bekanntmachung des Hinweises auf die Patenterteilung: **08.11.2023**
(21) Anmeldenummer: 15762475.0
(22) Anmeldetag: 06.07.2015
(51) Int. Cl.: A61B 5/107

(54) **ABBILDUNGSANORDNUNG**
IMAGING ARRANGEMENT
ENSEMBLE DE REPRÉSENTATION

(30) Priorität: 24.07.2014 DE 102014110431
(43) Veröffentlichungstag der Anmeldung: 31.05.2017
(73) Patentinhaber: Zebris Medical GmbH, 88316 Isny im Allgäu (DE)
(72) Erfinder: BRUNNER, Wolfgang, 88316 Isny im Allgäu (DE)
(74) Vertreter: Meissner Bolte Partnerschaft mbB
(86) Internationale Anmeldenummer: PCT/DE2015/200399
(87) Internationale Veröffentlichungsnummer: WO 2016/012018

(56) Entgegenhaltungen:
- WO-A1-2004/037085
- WO-A1-2014/081107
- AT-A4- 508 115
- DE-A1- 10 025 922
- DE-C1- 3 712 958
- DE-U1-202006 014 657
- US-A- 5 790 256
- US-A1- 2013 070 060
- US-A1- 2014 155 786
- US-B1- 6 546 356

## Beschreibung

Die Erfindung betrifft eine Abbildungsanordnung zur winkelgetreuen und tiefenscharfeh Abbildung und optionalen 3D-Vermessung räumlicher Objekte,

Traditionell werden sowohl räumliche als auch im Wesentlichen flächige Objekte (wie etwa Druckerzeugnisse) mit fotografischen oder Videokameras abgebildet, wobei in den letzten Jahrzehnten elektronische Bildsensoren die früher eingesetzten Fotofilme ersetzt haben. Mit zunehmender Pixelzahl und Fläche der Bildsensoren haben sich zunehmend hochwertige Abbildungen durch solche elektronischen Kameras erzielen lassen. Speziell bei der Aufnahme kleinerer Objekte lässt sich mit diesen Kameras auch eine relativ verzerrungsfreie Abbildung realisieren; größere Objekte oder solche mit großer Tiefen-Erstreckung lassen sich jedoch mit herkömmlicher Kameratechnik nur sehr bedingt winkelgetreu und tiefenscharf aufnehmen.

Zur Aufnahme flächiger Objekte haben sich seit Jahren so genannte Scanner etabliert, mit denen eine Vorlage abtastend aufgenommen wird. Am Markt erhältlich sind 2D Scanner auf der Basis von Contact Image Sensoren (CIS). Dies sind meist CMOS basierte Zeilensensoren, die mechanisch unter einer Glasplatte bewegt werden. Neben der notwendigen mechanischen Verfahreinheit haben die Sensoren den Nachteil, dass sie keine Tiefenschärfe besitzen und somit nur direkt in Kontakt befindliche Gegenstände vermessen können. Eine größere Tiefenschärfe haben Scannen basierend auf Zeilenkameras, die jedoch ebenfalls mechanisch, ggf. über ein Spiegelsystem, bewegt werden müssen. Darüber hinaus dauert eine Messung durch das mechanische Verfahren zumindest mehrere Sekunden. Damit sind nur statische Messungen möglich.

Schnellere Bildfolgen können mit bekannten 2D Scannervorrichtungen erzielt werden. Bei einem im Sportschuhhandel benutzten Gerät ist eine Glasplatte in einem Gehäuse mit integrierter Kamera untergebracht. Um eine Fuß- /Schuhgröße richtig bestimmen zu können, ist es aber nicht ausreichend, nur die Kontaktfläche zwischen Fuß und Glasplatte zu vermessen. Vielmehr ist der maximale Fersenabstand, nach hinten von Interesse. 3D Fußscanner arbeiten teilweise auf dem Prinzip der Lasertriangulation, benötigen aber auch einen großen Raum zur Unterbringung der Messeinheit. Übliche Scanner benötigen mechanische Verfahreinheiten, haben eine große Bauhöhe und lassen sich somit nicht - was für begleitende Analysen des Ganges vorteilhaft wäre - in flache Gehbahnen integrieren. Zum Stand der Technik gehören:

DE 37 12 958 C1, US 6 546 356 B1, WO 2004/037085 A1, DE 20 2006 014657 U1, AT 508 115 A4 welche allesamt Objekte vermessen.

Der Erfindung liegt daher die Aufgabe zu Grunde, eine Abbildungsanordnung der genannten Art bereitzustellen, die sich durch niedrige Kosten, Eignung für Vermessungsaufgaben und geringen Zeitbedarf für die Erzeugung des Gesamtbildes eines räumlichen Objektes auszeichnet.

Diese Aufgabe wird durch eine Abbildungsanordnung mit den Merkmalen des Anspruchs 1 gelöst. Zweckmäßige Fortbildungen des Erfindungsgedankens sind Gegenstand der abhängigen Ansprüche.

Es wird mit der Erfindung vorgeschlagen, eine Vielzahl von Kameraeinheiten (bevorzugt umfassend einen Sensorchip und eine diesem zugeordnete Abbildungsoptik) fest unterhalb eines abzubildenden Objektes - etwa auf einer Leiterplatte oder sonstigen Kameraträgerplatte - matrixförmig so anzuordnen, dass die Teil-Aufnahmebereiche aller Kameraeinheiten ein lückenloses zweidimensionales Gesamtbild ergibt. Eine geeignete Bildsyntheseeinheit dient dazu, aus den von den feststehenden Kameraeinheiten erzeugten Tellbildern ein Gesamtbild des Objekts im Aufhahmebereich der Anordnung zu synthetisieren.

Um die Bauhöhe der Messplatte möglichst niedrig zu halten, soll der Abstand zwischen den optischen Linsen und der Glasplatte möglichst gering sein, Deshalb ist es von Vorteil, die Kameras mit Weitwinkelobjektiven auszustatten. Da die Messvorrichtung keine mechanisch bewegten Teile aufweist, kann in schneller Messfolge dynamisch z.B. bei einem Abrollvorgang des Fußes eines Probanden gemessen werden.

Die durchsichtige Auflageplatte (Messfläche) wird praktischerweise von unten über matrixförmig angeordnete Leuchtelemente, vorzugsweise Leuchtdioden mit weißem Licht, beleuchtet. Dies erlaubt eine farbgetreue Wiedergabe des zu Scannenden Körpers. In einer besonderen Ausführung wird das zu vermessende Objekt (z.B. ein Fuß) mit Licht anderer Farbe beleuchtet, um bestimmte Konturen oder Läsionen der Haut besonders hervorzuheben und Defekte zu diagnostizieren. Vorzugsweise sind die Leuchtelemente als in der Lichtfarbe steuerbare RGB LEDs ausgeführt. Es können aber auch ausschließlich Weißlicht-LEDs oder auch sichtbares Licht emittierende LEDs in Kombination mit Infrarot (IR-LEDs) vorgesehen sein.

In einer Ausführung ist die Beleuchtungseinriclitung hinsichtlich der Beleuchtungs-Lichtfarbe einstellbar ausgeführt und umfasst, insbesondere mehrere wahlweise aktivierbare Gruppen von Leuchtelementen, die untereinander gleiche, aber zwischen den Gruppen unterschiedliche Lichtfarbe haben. Speziell ist hierbei eine Teilbild-Auswahlsteuerung zur Auswahl bestimmter Teilbilder von Kameraeinheiten zur Gewinnung verschiedener Gesamtbilder aus den Teilbildern von Kameraeinheiten eines bestimmten Typs vorgesehen.

In einer besonderen Ausführungsform wählt eine angeschlossene Recheneinheit automatisch die Farbe aus, die z.B. den besten Kontrast ergibt, Zur Realisierung dieser optimierten Beleuchtung ist speziell eine eingangsseitig mit der Bildsyntheseeihheit und ausgangsseitig mit der Beleuchtungseinheit verbundene Qualltätsprüf-/Steuereinheit vorgesehen, die zur selbsttätigen Ansteuerung einer Folge von Abhildungsvorgängen bei unterschiedlicher Lichtfarbe mit jeweils zugeordneter Bildsynthese, zur Auswertung der jeweils aufgenommenen Bilder hinsichtlich der ,Bildqualität und zur Auswahl einer diesbezüglich optimalen Lichtfarbe der Beleuchtungseinheit ausgebildet ist.

Wie bereits oben erwähnt, sind in einer Ausführungsform die (oder zumindest einige) Leuchtelemente als Infrarot LEDs ausgeführt, Damit können beispielsweise in der Haut befindliche Blutgefäße detektieret werden und insbesondere diagnostische Rückschlüsse in Hinsicht auf die Hautdurchblutung oder Verdickungen der Haut gezogen werden. Beim Einsatz des Scanners bei der Anpassung von Schuhen ist es besonders interessant, die Personen nicht nur barfuß, sondern auch mit farbigen aber auch schwarzen Socken zu vermessen. Auch hierbei kann mit IR Beleuchtung ein gutes Ergebnis erzielt werden.

Außer in einer matrixförmigen Anordnung können die Leuchtelemente auch nur am Rand der Messfläche angeordnet sein, oder es kann das Licht direkt über die seitlichen Kanten in die Auflageplatte (Glasplatte) eingekoppeft werden.

In einer weiteren Ausführungsform ist die Anordnung als dreidimensionaler Scanner zur Vermessung der Oberflächenform z.B. von Füßen unter Belastung ausgeführt. Hierbei ist es notwendig, dass mindestens zwei Kameras gleichzeitig dasselbe Messfeld beobachten. Eine Kamera nimmt das Muster aus einem anderen Blickwinkel auf und berechnet aus der Verzerrung die Distanzen. Das Bild der Gesamtoberfläche (das nicht notwendigerweise ein Gesamtbild im eigentlichen Sinne sein muss und daher hier auch als Bereichsbild bezeichnet wird) wird aus den einzelen von den beteiligten Kameraeinheiten in ihren überlappenden Teiibildern gelieferten Teiloberflächen zusammengesetzt und gegebenenfalls rechnerisch geglättet. Hierbei wird die Struktur des zu vermessenden Objekts (bei menschlichen Körperteilen z.B. Haut oder Bekleidung) zur korrekten "Montage" der Teilbilder zur Erzeugung des Bereichsbildes verwendet.

Unter Vorrichtungsaspekten ist bei dieser Ausführung der Bildsyntheseeinheit eine Teilbild-Auswahlsteuerung zur Auswahl bestimmter Teil bilder von Kameraeinheiten zur Gewinnung verschiedener Bereichsbilder aus jeweils mehreren den gleichen Objektbereich darstellenden Teilbildern von Kameraeinheiten mit überläppendem Aüfnahmebereich zugeordnet.

In einer besonderen Ausführungsform ist die Beleuchtungseinrichtung bzw, sind speziell die üblicherweise vorhandenen Leuchtelemente oder auch zusätzliche Leuchtelemente so beschaffen, dass sie ein definiertes Muster auf das Objekt projizieren können. In einer weiteren Ausgestaltung ist die Auflageplatte an der Ober- und/oder Unterseite mit einem Muster versehen . Dies kann beispielsweise ein Streifenmuster, ein Grid aus sich kreuzenden Linien oder ein reguläres Punktmuster sein. Dieses Muster kann von den Leuchtelementen auf das Objekt projiziert werden und ermöglicht so die 3D-Oberfiächenberechnuhg und gegebenenfalls eine Vermessung. In einer weiteren Ausgestaltung wird der Grid zur Kalibrierung des zweidimensional oder dreidimensional messenden Scanners verwendet. Damit können die Bilder der Kameraeinheiten über einen Kalibriervorgang entzerrt werden. Damit kann auch die Ablenkung oder eine sonstige Verfälschung des Strahlengangs durch die Glasscheibe der Auflageplatte kompensiert werden.

Die Ansteuerung der matrixförmig angeordneten Kameraeinheiten kann sequentiell, zeilen- oder spaltenweise erfolgen, in einer bevorzugten Ausführung haben die Kameraeinheiten einen Bildspeicher, sodass mehrere Kameras gleichzeitig ein Bild belichten können und diese nacheinander ausgelesen werden. Die Bilddaten können dann über eine geeignet ausgebildete Schnittsteile in einen externen Rechner übertragen und weiter bearbeitet werden. Insbesondere ist der Bildsyntheseeinheit eine Bildauswertungs- /Vermessungseinheit oder eine Schnittstelle zum externen Anschluss einer solchen zugeordnet

In einer weiteren Ausführungsförm der Erfindung ist die vorgeschlagene Abbildungsanordnung mit einem kastenförmigen Gehäuse versehen, das eine die Auflageplatte haltende und zugleich eine Kamerakammer unterhalb der Aufiageplatte und eine Objektkammer oberhalb der Auflageplatte begrenzende, die Kamerakammer und Objektkammer gegenüber Fremdlicht abschirmende Seitenwand hat.

In einer weiteren Ausführung sind die Kameraeinheiten auf einer gemeinsamen Kameraträgerplatte angebracht, welcher mindestens ein Aktor zur Bewegung der Kameraträgerplatte und somit zur gemeinsamen und gleichzeitigen Bewegung aller auf dieser angebrachten Kameraeinheiten in mindestens einer Raumrichtung zugeordnet ist. Speziell ist hierbei dem oder jedem Aktor eine Kamerabewegungs-Steuereinheit zugeordnet, welche über eine Synchronisierungseinheit mit einem Eingang der Bildsyntheseeinheit verbunden ist. Hierbei sind speziell die Kamerabewegungs-Steuereinheit und Bildsyntheseeinheit derart ausgebildet, dass die Gewinnung des Gesamtbildes des Objekts in Abhängigkeit von der Bewegung bzw. den Positionen der Kameraträgerplatte während einer Bewegung erfolgt.

Die Aktoren können nach dem Wirkungsprinzip beispielsweise auf magnetischer oder piezoelektronischer Basis arbeiten oder in klassischer Weise über Treibriemen- oder Spindelverfahreinheiten oder anderen Vorschubeinheiten auf die Kameraträgerplatte oder gegebenenfalls auch nur Teil-Platten einwirken. Dies kann in der Ebene der Kameraträgerplatte (xy-Ebene), aber auch in der Tiefe (z-Ricritung) erfolgen, um z.B. andere optische Eigenschaften der Kameraeinheiten zu erhalten.

In einer besonderen Ausgestaltung wird die Kameraträgerplatte nur um einen kleinen Abstand, z.B. um einen halben oder ganzen Kameramatrixabstand, verfahren. Damit kann die Messauflösung erhöht bzw. die Anzahl der benötigten Kameras verringert werden. Insbesondere kann beispielsweise das System in Grundposition zweidimensional messen. Durch den kleinen Verfahrschritt der Aktoren kann eine zweite oder dritte Aufnahme aus einer anderen Perspektive erfolgen, um eine dreidimensionale Vermessung der Oberfläche vorzunehmen.

In einer anderen Ausführungsförm wird in der Grundposition einer verfahrbaren Kamefaträgerplatte mit den hierauf fest angebrachten Kameraeinheiten nur ein Teil der gesamten Messfläche (des Aufnahmebereiches) abgebildet, Durch einen größeren Verfahrbereich der Kameraträgerplatte können dann praktisch beliebig große Flächen nacheinander erfasst werden. In einer weiterführenden Ausgestaltung dieses Konzepts ist es möglich, die Abbildungsanordnung aus in einer einzelnen Zelle angeordneten Kameraeinheiten und einem uniaxialen Aktor mit derart großem Verfahrbereich zu bilden, dass mit dieser einzelligen Anordnung der gesamte gewünschte Aufnahmebereich realisiert werden kann.

Die (einzeilige) Kameraträgerplatte wird dann nur in eine Vorschubrichtung bewegt, wobei die Einzelbilder entweder kontinuierlich oder in einzelnen Schritten aufgenommen werden. Auch hier sind zwei- und dreidimensionale Aufnahmen der Oberfläche des Messkörpers möglich,

In einer weiteren Ausführungsform ist es möglich, Farbkameramodule und infrarotempfindliche s/w Kameramodule auf der Messmatrix gemischt anzuordnen. Unter Rückgriff auf das vorstehend erläuterte Konzept ist es beispielsweise möglich, eine Zeile mit Farbkameras und eine weitere parallele Zeile mit s/w Kameras zu bestücken und diese gleichzeitig zu verfahren.

In weiteren Ausführungen der Erfindung sind der Auflageplatte Druckerfassungsmittel zur, insbesondere ortsaufgelösten, Erfassung des durch das Objekt auf die Auflageplatte ausgeübten Drucks zugeordnet. Die Druckerfassungsmittel lassen sich in vielgestaltiger Weise realisieren, solange sichefgestellt ist, dass die Auflageplatte der Abbildungsanordnung vollständig oder auch nur bereichsweise mindestens so weit durchsichtig bleibt, dass eine zufriedenstellende Abbildung des Objekts gewährleistet ist.

Neben herkömmlichen Druck- bzw. Kraftsensoren kann hierfür auch beispielsweise eine optische Erfassung von druckbedingten Verformungen eines vorgegebenen Musters auf der Auflageplatte durch die Kameraeinheiten selbst, mit nachgeordneter entsprechender Auswertung der aufgenommenen Bilder, vorgesehen sein. Bei dieser Variante ist auf der Auflageplatte eine Musterplatte vorgesehen, die ein druckverformbates Muster, beispielsweise eine Matrixanordnung elastisch verformbarer Pyramiden, zur ortsaufgelösten optischen Erfassung des durch das Objekt auf die Auflpgeplatte ausgeübten Drucks aufweist. Genauer gesagt, ist vorgesehen, dass die Druckerfassungsmittel durch die Musterplatte mit druckverformbarem Muster und eine zur Ausweftung von Verformungen des Musters ausgebildete Muster-Auswertungseinheit gebildet sind, die der Bildsyntheseeinheit nachgeordnet ist.

In einer weiteren Ausführung der Druckerfassungs-Option weisen die Druckerfassungsmittel eine auf die Auflageplatte aufgebrachte, mindestens im überwiegenden Flächenanteil der Auflageplatte durchsichtige Drucksensormatrix, z.B. mit kapazitiven Drucksensoren, auf, Insbesondere kann hier eine kapazitive Sensormatrix vorgesehen sein, die aus sich kreuzenden schmalen leitfähigen Bändern gebildet ist. In den Kreuzungspunkten ist ein elastisches Dielektrikum eingefügt, das bei Druck den Plattenabstand und damit die Kapazität ändert. Die Aussparungen ergeben eine freie "Sicht" auf die Glasplatte, wodurch die Scännerfunktion erhalten bleibt. In einer anderen Variante beinhaltet die Sensormatrix ein durchsichtiges elastisches Dielektrikum. Die Leiterbahnen sind durchsichtig leitfähig und z.B. metallisch aufgedampft, Anstelle des elastischen Dielektrikums kann auch ein starres Dielektrikum mit Aussparungen verwendet werden, in die sich die Leiterbahnen unter Belastung einwölben können.

Alternativ zum Vorsehen einer Drucksensormatrix auf der Auflageplatte können unter der Auflageplatte einzelne Drucksensoren angeordnet sein, welche die Auflageplatte mindestens teilweise abstützen. Auch eine Kombination einer ortsaufgelöst messenden Drucksensormatrix mit derartigen abstützenden Drucksensoren zur summarischen Erfassung des auf die Auflageplatte oder größere Bereiche derselben wirkenden Drucks ist möglich.

Sinnvollerweise ist beim Vorsehen von Druckerfassungsmitteln die Abbildurigsanordnung zusätzlich mit einer Drucksignal-Auswertungsoinheit oder einer Schnittstelle zum externen Anschluss einer solchen versehen, die insbesondere Mittel zur Synchronisierung mit der Bildsyntheseeitiheit zur synchronisierten Gewinnung eines optischen Gesamtbildes und Druckverteilungs-Bildes eines Objekts aufweist.

Die vorgeschlagene Abbildungsanordnung kann vielgestaltige Anwendung finden, aus derzeitiger Sicht der Anmelderin insbesondere bei der abbildenden Untersuchung und Vermessung der Füße eines Menschen im Rahmen orthopädischer Untersuchungen oder zur Anpassung von Sportschuhen oder anderer Fußbekleidung, oder auch des Kopfes, zur Anpassung von Helmen o.ä. Unabhängig von Körperteil-Abbildungen und zugehörigen Vermessungsaufgabeh ist sie auch für sonstige anspruchsvolle Abbildungsaufgaben geeignet, die mit einer herkömmliehen (auch hochwertigen) Kamera oder einem handelsüblichen 2D-Scanner grundsätzlich nicht zufriedenstellend gelöst werden können,

Speziell im Zusammenhang mit der Untersuchung von Füßen ist auch der Einsatz in einer Laufbendanordnung möglich, um zugleich Aussagen zum Gang bzw, Laufstil des Probanden zu gewinnen. Entsprechend liegt im Bereich der Erfindung auch eine Laufbandanordnung zur Überwachung und/oder zum Training des Gehens oder Laufens, mit einer Abbildungsanordnung nach einem der vorangehenden Ansprüche mit den oben erläuterten Merkmalen und einem durchsichtigen Laufband. Die Abbildungsanordnung ist unterhalb einer Lauffläche des Laufbandes angeordnet, und das durchsichtige Laufband wird über die Auflageplatte der Abbildungsanordnung bewegt oder bildet selbst eine bewegliche Aufiageplatte.

Vorteile und Zweckmäßigkeiten der Erfindung ergeben sich im Übrigen aus der nachfolgenden skizzenartigen Beschreibung von Ausführungsbeispielen anhand der Figuren, Von diesen zeigen:
Fig. 1 eine skizzenartige Gesamtansicht einer Abbildungsanordnung gemäß einer Ausführungsform der Erfindung,
Fig. 2 eine skizzenartige Gesamtansicht einer Abbildungsanordnung gemäß einer weiteren Ausführungsform der Erfindung, kombiniert mit einer Druckerfassungs- und -Auswertungseinrichtung,
Fig. 3 eine schematische Ausschnittsdarstellung eines Arrays von Kameraeinheiten der Abbildungsanordnung nach Fig. 1,
Fig. 4A und 4B schematische Längsschnittdarstellungen des optischen Teils der Abbildungsanordnung nach Fig. 1, mit hinsichtlich ihres Teil-Erfassungsbereiches unterschiedlich ausgeführten Kameraeinheiten,
Fig. 5 eine skizzenartige Darstellung zur Erläuterung einer weiteren Ausführung der Erfindung,
Fig. 6 eine skizzenartige perspektivische Darstellung einer weiteren Ausführung der Erfindung, mit einer speziell ausgebildeten Auflageplatte,
Fig. 7 ein schematisches Blockschaitbild zur Erläuterung einer weiteren Ausgestaltung der Erfindung,
Fig. 8A und 8B eine schematische Schnittdarstetlung bzw. Draufsicht der Druckerfassungsmittel einer weiteren Ausführung der Erfindung,
Fig. 9 eine schematische Darstellung einer weiteren Realisierung der Druckerfassungsmittel,
Fig. 10A und 10B eine schematische Darstellung einer weiteren Realisierung der Druckerfassungsmittel,
Fig. 11 eine schematische Seitenansicht des Laufbands einer Laufbandanordnung mit integrierter erfindungsgemäßer Abbildungsanordnung,
Fig. 12 eine Ausführung der Abbildungsanordnung mit bewegbarer Kameraträgerplatte und
Fig. 13 eine Modifikation der Ausführungen nach Fig. 12 mit zeilenartiger und uniaxial bewegbarer Kameraträgerplatte.

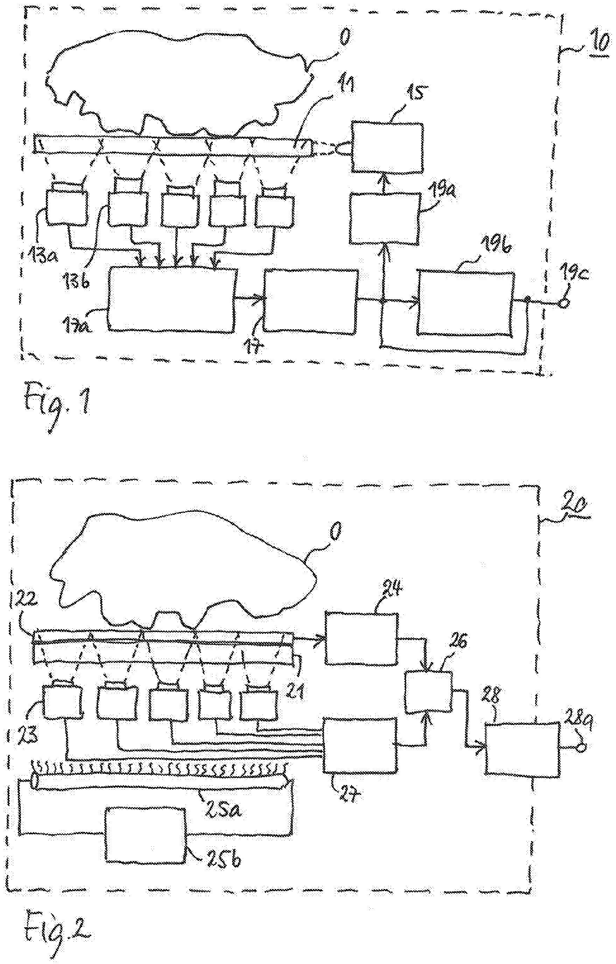

Fig. 1 zeigt in Art eines Blockschaltbildes schematisch eine erfindungsgemäße Abbildungsanordnung 10 zur winkelgetreuen und tiefehscharfen Abbildung eines Objekts O, welches auf einer Auflageplatte 11 angeordnet ist. Unterhalb der Auflageplatte 11 ist, in matrixförmiger Anordnung eine Mehrzahl von Kamereinheiten 13a, 13b zweier verschiedener Kameratypen angeordnet, nämlich im Bereich des sichtbaren Lichts aufnehmende Kameraeinheiten 13a und Infrarot-Kameraeinheiten 13b. Zur Beleuchtung des Objekts Q ist eine Beleuchtungseinrichtung 15 vorgesehen, die das von ihr erzeugte Licht (in diesem Fall bevorzugt weißes Licht mit IR-Anteilen) in die Seitenkanten der als Auflogeplatte dienenden Glasplatte 11 einkoppelt und somit eine über die Fläche der Glasplatte 11 homogene Objektbeleuehtung realisiert, Die Kameraeinheiten 13a, 13b haben jeweils einen Aufnahmebereich, mit dem ein Teil des Objekts erfasst wird, und die Teil-Aufnahmebereiche setzen sich zu einem Gesamt-Aufnahmebereich der Abbildungsanurdnung zusammen.

Den Kameraeinheiten 13a, 13b ist bei dieser Ausführung eine Teilbild-Auswahlsteuerung 17a als vorgelagerte Funktionseinheit einer Bildsyntheseeinheit 17 zur Synthese eines Gesamtbildes des Objekts aus den von den Kameraeinheiten gelieferten Teilbildern nachgeordnet In der Teilbild-Auswahlsteuerung erfolgt die Auswahl bestimmter Teilbilder der Kameraeinheiteh 13a, 13b, im vorliegenden Beispiel insbesondere entweder der Teilbilder der im sichtbaren Licht arbeitenden Kameraeinheiten 13a oder der IR-Kameraeinheiten 13b zur Erzeugung eines mit sichtbarem Licht aufgenommenen oder im IP-Bereich aufgenommenen Gesamtbildes. Der Bildsyntheseeinheit 17 sind zwei Auswertungseinheiten 19a, 19b unterschiedlicher Funktion nachgeschaltet, und zwar eine Blldgualitäts Auswertungseinheit 19a zur Auswertung der Bildqualität bei unterschiedlichen Einstellungen der Beleuchtungseinrichtung 15 und zur Vorgabe einer optimalen Beleuchtungssteuerung und eine Bildauswertungs-/Vermessungseinheit zur Vermessung des Objekts O anhand des aufgenommenen Gesamtbildes (oder auch mehrerer Gesamtbilder in unterschiedlichen Wellenlängenbereichen). Schließlich hat die Abbildungsanordnung 10 einen Ausgangsanschluss 19c zur Ausgabe der Bilddaten und Vermessungsergebnisse an externe Einheiten zur weiteren Auswertung, Speicherung oder Übersendung an entfernte Nutzer,

Fig. 2 zeigt eine ähnliche Abbildungsanordnung wie Fig. 1, hier bezeichnet mit Ziffer 20, Soweit diese gleiche oder im Wesentlichen funktionsgleiche Komponenten wie die Abbildungsanordnung 10 nach Fig. 1 aufweist, wurden diese mit an Fig. 1 angelehnten Bezugsziffern bezeichnet und werden hier nicht nochmals erläutert. Bei der Abbildungsanordnung 20 ist anstelle einer in die Seitenkante der Auflageplatte 21 einkoppelnden Beleuchtungseihrichtung eine unterhalb oder seitlich unterhalb von den Kameraeinheiten 23angeordnete Lichtquelle 25a mit Beleuchtungssteuerung 25b vorgesehen (etwa eine oder mehrere Gasentladungslampen oder ein LED-Array). Die Kameraeinheiten 23 sind hier identisch und durch im sichtbaren Bereich empfindliche Bildsensoren mit zugeordneter (nicht separat gezeigter) Abbildungsoptik gebildet. Innen. ist hier eine Bildsyntheseeinheit 27 unmittelbar nachgeschaltet.

Zusätzlich umfasst die Anordnung 20 eine auf die Auflageplatte 21 aufgelegte Druckerfassungsmatrix 22, die zur ortsaufgelösten Erfassung des durch das Objekt O auf die Auflageplatte ausgeübten Drucks mit matrixförmig angeordneten Ducksensoren ausgestattet ist. Die Druckerfassungsplatte 22 kann einen an sich bekannten Aufbau haben. Es ist aber für die Abbildungsanordnung 20 funktionswesentlich, dass sie durch geeignete Ausführung der Drucksensoren entweder insgesamt durchsichtig ist oder jedenfalls hinreichend große und gleichmäßig verteilte durchsichtige Bereiche aufweist, um eine angemessene Erfassung des Objekts durch die Kameraeinheiten 23 sicherzustellen.

Die Druckerfassungsplatte 22 ist mit einer Drucksignal-Auswertungseinheit 24 verbunden, die (beispielsweise mit einem an sich bekannten Aufbau) zur Auswertung der Signale der in der Druckerfassungsplatte 22 enthaltenen punktuellen Drucksensoren ausgebildet ist. Eine Synchronisationsstufe 26 stellt sicher, dass die Auswertungsergebnisse der Drucksignal-Auswertungseinheit 24 und die von der Bildsyntheseeinheit 27 gelieferten Bilder des Objekts, beispielsweise in unterschiedlichen Lagen und/oder Zuständen externer Belastung des Objekts zueinander korrespondieren, und sie liefert synchronisierte Bild- und Druckdaten an eine Ausgabeschnittstelle 28 mit zugeordnetem Ausgangsanschluss 28a.

Es wird darauf hingewiesen, dass die Abbildungsanordnungen nach Fig. 1 und 2 in vielgestaltiger Weise modifiziert und Teilaspekte beider Anordnungen miteinander kombiniert werden können, ohne dass damit der Bereich der Erfindung verlassen würde. Auch das Fortlassen einzelner Funktionen oder Komponenten ist im Rahmen der Erfindung möglich,

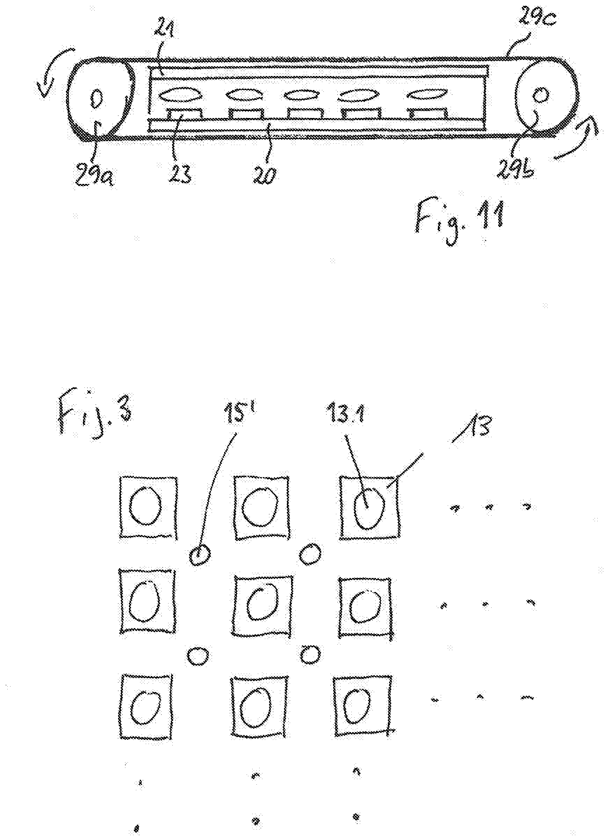

Fig. 3 zeigt schematisch eine matrixförmig verschachtelte Anordnung von Kameraeinheiten 13 mit zugeordneter Abbildungsoptik 13.1 und LEDs 15' auf einer gemeinsamen Kamera-/LED-Trägerplatte 14 zur Realisierung sowohl der Aufnahme-Komponente als auch der Beleuöhtungseinrichtung einer erfindungsgemäßen Abbildungsanordnung, Die LEDs 15' sind hier jeweils "diagonal" zwischen Eckbereichen der Kameraeinheiten 13 angeordnet, sie können aber auch jeweils zeilen- und spaltenmäßig ausgerichtet mit diesen platziert sein, Es ist auch nicht erforderlich, dass zwischen allen benachbarten Kameraeinheiten jeweils eine LED angeordnet ist, sondern es können auch LEDs jeweils lediglich in ausgewählten Abstandsbereichen der Kameraeinheiten platziert sein.

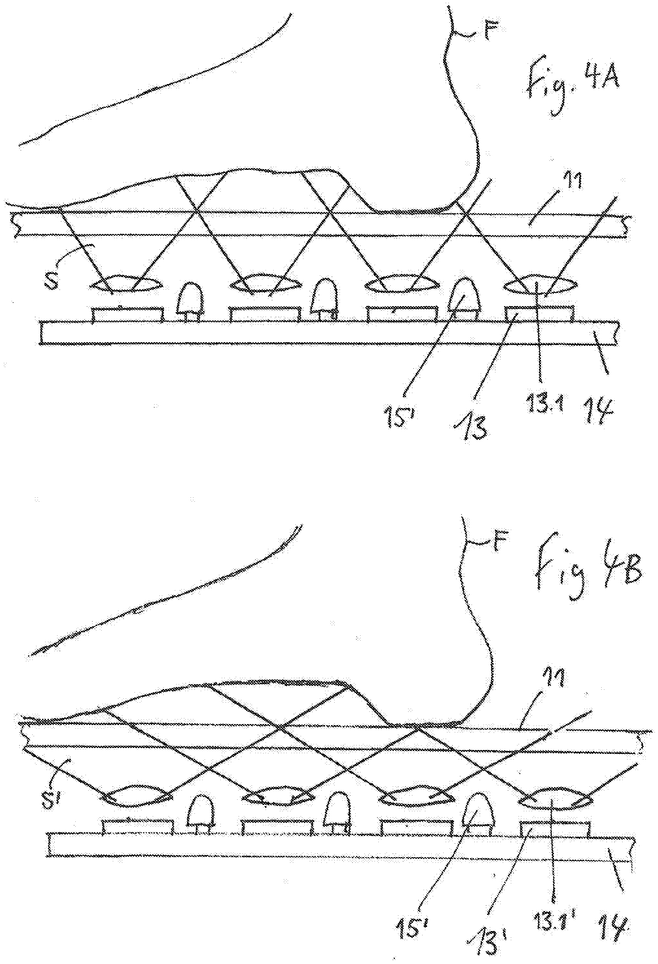

Fig. 4A und 4B zeigen in schematischen Längsschnittdarstellungen jeweils, wie ein Fuß F eines Menschen (als spezielles Objekt) auf einer Auflageplatte 11 von Kameraeinheiten 13 mit Abbildungsoptik 13.1 (Fig. 4A) bzw. Kameraeinheiten, 13' mit Abbildungsoptik 13.1' (Fig. 4B) abgebildet wird. Es ist jeweils der Teil-Aufnahmebereich S (Fig. 4A) bzw. S' (Fig. 48) der Kameraeinheiten schematisch dargestellt. Bei der Ausführung nach Fig. 4A schließen die Aufnahmebereiche S auf der Oberseite der Auflageplatte 11 lückenlos, aber ohne wesentliche Überlappung aneinander an, während bei der Ausführung nach Fig. 48 sich die Aufnahmebereiche S' benachbarter Kameraeinheiten 13' so weit überlappen, dass der Aufnahmebereich jeder Kameraeinheit von den jeweils benachbarten Kameraeinheiten jeweils gemeinsam ein zweites Mal vollständig abgedeckt wird,

Mit der Anordnung nach Fig. 4B wird insbesondere die weiter oben erläuterte 3D-Abtastung des Fußes, speziell unter Belastung, realsiert. Die zugehörige Signaiverarbeitung überlappender Teilbilder, die aus unterschiedlichen Aufnahmepositionen aufgenommen wurden, ist dem Fachmann der Bildverarbeitung an sich bekannt und bedarf daher hier keiner genaueren Erläuterung.

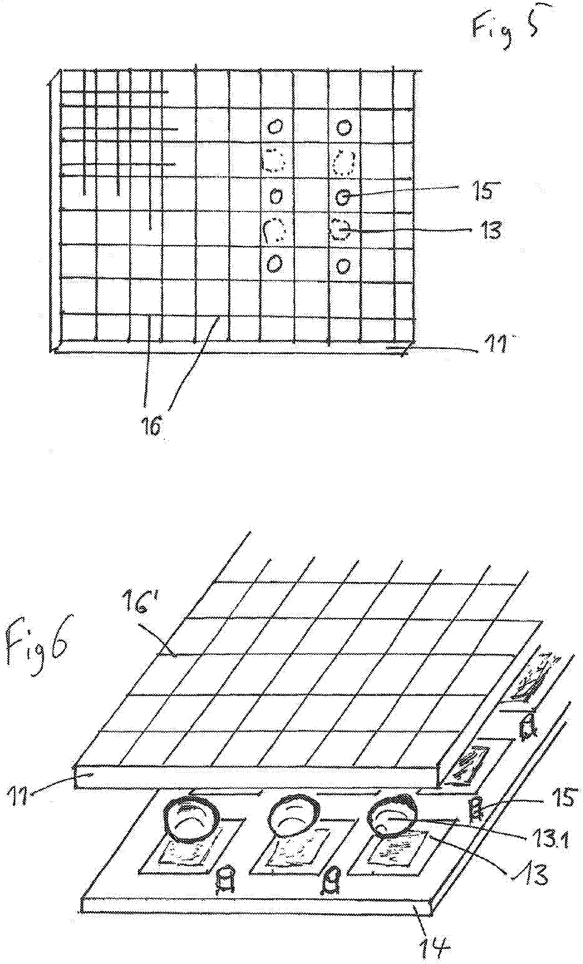

Fig. 5 zeigt schematisch ein auf einer Auflageplatte 11 durch Projektion mittels geeigneter Beleuchtungselemente 15 erzeugtes Projektions-Gitter 16. Das Projektions-Gitter 16 kann, wie in Fig. 6 skizzenartig gezeigt, durch ein durch die Auflageplatte 11 aufgetragenes (zum Beispiel aufgedrucktes) Muster (Grid) 16' ersetzt sein. Eine weitere Modifikation kann darin bestehen, dass auf eine ungemusterte Aufiageplatte 11 eine Musterplatte oder -folie aufgelegt ist. Dies hat den Vorteil, dass die Abbildungsanordnuhg leicht und kostengünstig mit verschiedenen Mustern zur Unterstützung der Abbildung unterschiedlicher Objekte oder zu unterschiedlichen Zwecken versehen werden kann.

Die auf die aufzunehmende Objektoberfläche projizierten Muster können insbesondere anstelle objekteigener Strukturen oder sonstiger objekteigener zuordnungsmerkmale bei der Synthese des Gesamtbildes oder eines Bereichsbiides aus von einzelnen Kameraeinheiten aufgenommenen Teilbildern zur korrekten "Montage" der Teilbilder benutzt werden. Des Weiteren können sie bei druck-verformbaren Objekten zur Aufzeichnung von Verformungs-Situationen und gegebenenfalls auch zur Gewinnung von Aussagen über die einwirkenden Kräfte genutzt werden.

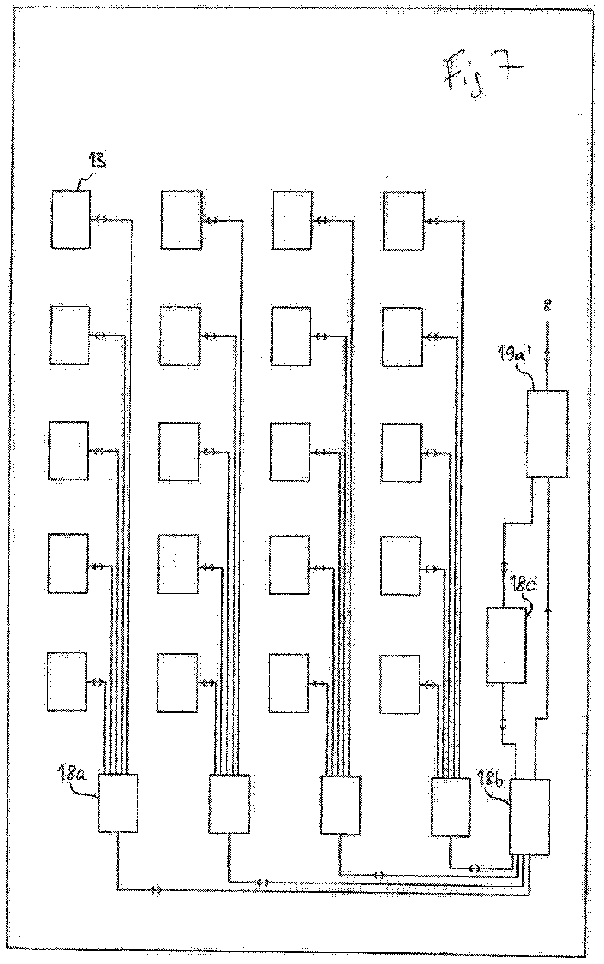

Fig. 7 zeigt schematisch, wie die matrixförmige Anordnung von Kameraeinheiten 13 jeweils spaltenweise (oder zeilenweise) über programmierbare Bausteine CPLDs 18a, 18b, 18c angesteuert und ausgelesen, werden kann, wie weiter oben bereits unter Verfahrensaspekten erwähnt, Hierbei gelangen die Bildsignale der Kameraeinheiten einer Spalte jeweils über einen Spalten-CPLD 18a zu einem Anordnungs-CPLD 18b und von dort über einen als USB-Anschluss ausgebildeten Ausgangsanschluss 19a' zu einem angeschlossenen Rechner oder anderen externen Gerät, während die Ansteuerung der Kameraeinheiten von außen über einen Controller-CPLD 18c erfolgen kann. Eingebaute (nicht gezeigte) Bildspeicher der Kameraeinheiten realisieren eine interne Bildsignalspeicherung, die einen sequentiellen Betrieb ermöglicht.

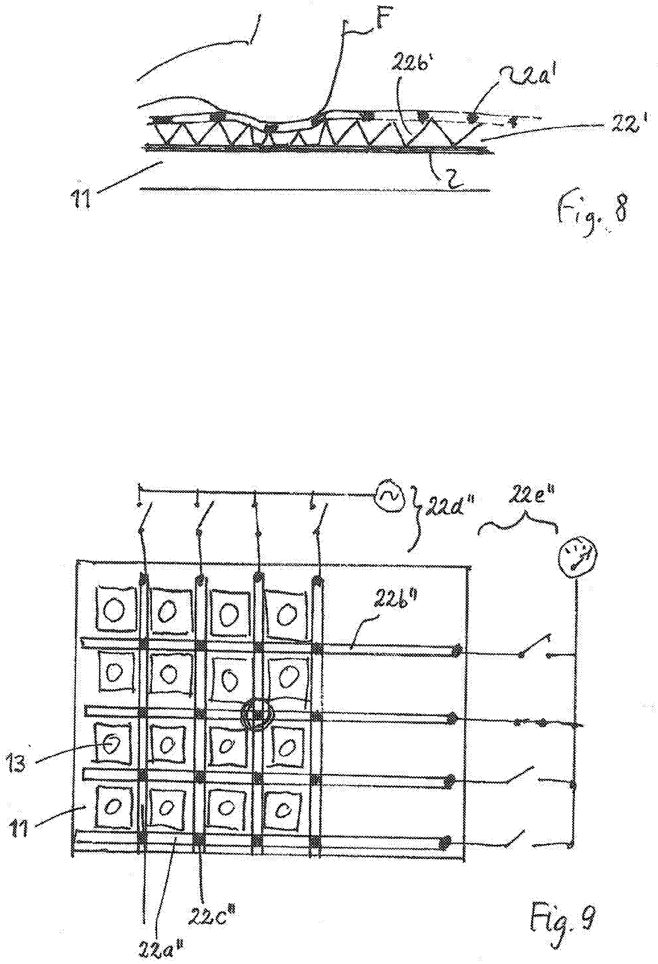

Fig. 8 zeigt in einer Längsschnittdarstellung (Detailansicht) eine Realisierungsmöglichkeit einer kombinierten Scan- und Druckaufnahmefunktion mittels einer auf die Auflageplatte 11 aufgelegten Spezialpiatte 22' zur lichtoptischen Druckerfassung. Die Spezialplatte 22' hat an ihrer (in der Abbildung oben liegenden) Basisschicht ein reguläres Muster 22a' und trägt eine reguläre Anordnung angespritzter Pyramiden 22b', die mit den Spitzen nach unten auf die Auflageplatte 11 zu liegen kommen. Unter Druckeinwirkung (beispielsweise mit aufgesetztem Fuß F) verformen sich die Pyramiden wie in der Figur dargestellt, was zugleich zu einer Verzerrung des von unten mittels der (hier nicht dargestellten) Kameraeinheiten aufgenommenen Abbildes des Musters 22a' führt. Der Grad der Verzerrung des Musters widerspiegelt bis zu einem gewissen Grade die Größe der durch den Fuß ausgeübten Druckkraft und kann daher als Maß für jene genutzt werden.

Aufgrund der generellen Trägheitsarmut, und hohen Abbildungsgeschwindigkeit der vorgeschlagenen Abbildungsanordnung lassen sich mit dieser Modifikation auch dynamische Messungen, also die Erfassung von Druckwechselbelastungen, ausführen. Wichtig für die Gewährleistung der Hauptfunktion der Anordnung, nämlich der präzisen Abbildung des Fußes F, ist eine hinreichende Transparenz der Spezialplatte 22'.

Fig. 9 zeigt schematisch eine weitere Realisierungsform einer zusätzlichen Druckerfassung bei der vorgeschlagenen Abbildungsanordnung. Auf die Auflageplatte 11 ist eine Matrix aus kapazitiven Drucksensoren aufgebracht, die sich kreuzende schmale leitfähige Bänder 22a", 22b" und an den Kreuzungspunkten ein zwischengelegtes elastisches Dielektrikum 22c" zur Bildung jeweils eines Sensorpunktes umfasst. Über spaltenweise Ansteuerung 22d" und zeilenweise Auslesung 22e" lassen sich die Kapazitätswerte der einzelnen Sensorpunkte (Leitungs-Kreuzungspunkte) und somit die dort jeweils wirkende Druckkraft auslesen.

Bei dieser Ausführung gewährleistet die relativ geringe Abschattung durch die schmalen Leiterbahnen einen nur geringfügig eingeschränkten Aufnahmebereich der Kameraeinheiten 13 und somit eine praktisch unbeeinträchtigte Abbildungsfunktion der Anordnung.

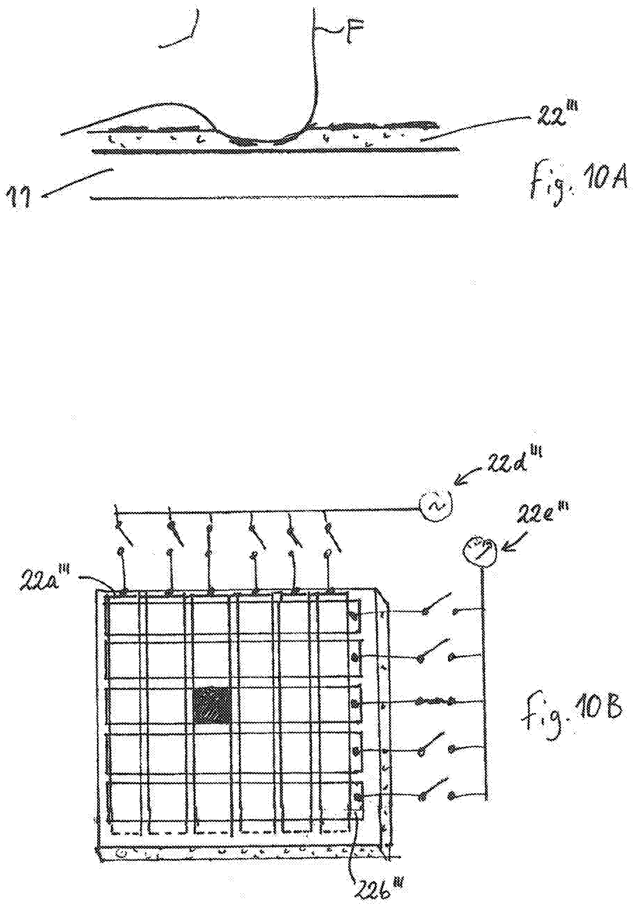

Fig. 10A und 10B zeigen eine ähnliche Anordnung wie Fig. 9, in einer schematischen Längsschnittdarstellung bzw. synoptischen Darstellung. Von der Anordnung nach Fig. 9 unterscheidet sie sich durch das Vorsehen einer vollflächig auf der Auflageplatte 11 aufliegenden, kapazitiv wirkenden Sensormatte 22‴, die aus einem druckelastischen durchsichtigen Dielektrikum besteht. Auch die Leiterbahnen 22a‴ und 22b‴ sind hier transparent und können beispielsweise als ITO-Bedampfung der Matte ausgeführt sein. Die Funktionsweise der einzelnen Sensoren und die Art und Weise von deren Ansteuerung und Auslesen ist vergleichbar der Anordnung von Fig. 9 und dem Fachmann grundsätzlich vertraut.

Fig. 11 zeigt schematisch, als Kernstück einer grundsätzlich bekannten Laufbandanordnung, ein über zwei Walzen 29a, 29b gezogenes Laufband 29c, unterhalb dessen der optische Teil einer Abbildungsanordnung 20 nach Fig. 2 (oder auch einer Abbildungsanordnung 10 nach Fig. 1 oder einer anderen erfindungsgemäßen Abbildungsanordnung) untergebracht ist. Bei einer solchen Anordnung stellen die Füße einer über das Laufband 29c laufenden Person jeweils im Moment des Laufbandkontakts das (nicht gezeigte) Abbildungsobjekt dar.

Es versteht sich, dass das Laufband 29c hinreichend durchlässig für die Strahlung der eingesetzten (hier ebenfalls nicht gezeigten) Beleuchtungseinrichtung sein muss, um eine hinreichende Abbildungsqualität trotz des zusätzlichen Vorhandenseins des Laufbandes zu gewährleisten. Grundsätzlich kann - bei hinreichend steifer Ausführung des Laufbandes - auch das Laufband selbst die Auflageplatte der Abbildungsanordnung bilden, so dass optische Verluste, wie sie unvermeidbar an jeder optischen Grenzfläche zwischen verschiedenen Komponenten auftreten, reduziert werden können.

Es ist auch möglich, die Laufbandanordnung mit der weiter oben erläuterten Fortentwicklung der Abbildungsanordnung zu bestücken, bei der eine zusätzliche otsaufgelöste Druckerfassung implementiert ist. Diese Kombination ist wegen der umfangreichen Aussagemöglichkeiten zum Gang bzw. Laufstil des Probanden besonders bevorzugt, weist allerdings eine relativ hohe Komplexität auf und erfordert nach derzeitigem Erkenntnisstand Kompromisse bei der Abbildungsqualität.

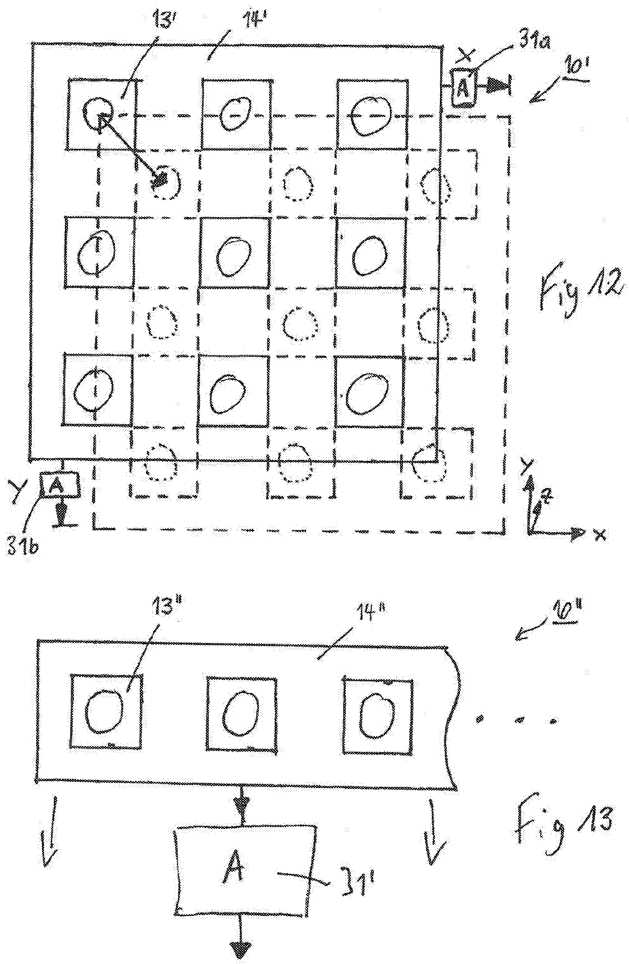

Fig. 12 zeigt schematisch als wesentliche Teile einer weiteren Abbildungsanordnung 10' eine Variante, bei der eine Kanieraträgerplatte 14'und matrixartige Anordnung von Kameraeinheiten 13' gemeinsam mittels Aktoren bzw. Antrieben (z.B, Piezoaktoren) 31a, 31b in einer Ebene parallel zur Erstreckungsebene der Kameraträgerplatte 14'in x- bzw. y-Richtung bewegbar sind. Mit durchgezogenen Linien bzw, gestrichelten Linien sind die beiden Aufnahmepositionen der Anordnung 10' dargestellt, die bei diesem Beispiel zur Erhöhung der Auflösung der Abbildung (bei gleichbleibender Anzahl der Kameraeinheiten) bzw, zur Ausführung einer 3D-Abbildung die geeignet ist, bei der ein in der einen Extremposition erzeugtes Bild mit einem in der anderen Extremposition erzeugten Bild des Objektes kombiniert wird.

Fig. 13 zeigt, als Kernstück einer weiter modifizierten Abbildungsanordnung 10" eine zeilenförmige Anordnung von Kameraeinheiten 13" auf einem entsprechend leistenförmigen Kameraträger 14", angetrieben durch einen uniaxialen Antrieb 31'. Der Antrieb 31' hat hier einen wesentlich größeren Verschiebebereich als bei der Ausführung nach Fig. 12 zu realisieren, da die Zeilenkameraanordnung in ihrem Verschiebebereich den gesamten Aufnahmebereich der Abbildungsanordnung 10" abdecken muss. Die einzelnen Bilder können entweder kontinuierlich während der Verschiebung oder in einzelnen Schritten in bestimmten Stopp-Positionen des Bewegungsvorganges aufgenommen werden. Auch bei dieser Ausführung sind 3D-Aufnahmen der Oberfläche des (nicht dargestellten) Objektes möglich.

Die Ausführung der Erfindung ist nicht auf diese Beispiele beschränkt, sondern wird durch die folgenden Ansprüche definiert.

## Patentansprüche

1. Abbildungsanordnung (10) zur winkelgetreuen und tiefenscharfen Abbildung und optionalen 3D-Vermessung räumlicher Objekte, mit einer durchsichtigen Auflageplatte (11) für ein abzubildendes Objekt, das auf der Auflageplatte platziert wird, einer auf die Auflageplatte ausgerichteten Beleuchtungseinrichtung (15) zur Beleuchtung des Objekts, einer Vielzahl von matrixförmig im Wesentlichen derart feststehend in einer gemeinsamen, zur Auflageplatte parallelen Ebene in vorbestimmtem gleichem Abstand zur Auflageplatte unterhalb der Auflageplatte angeordneten Kameraeinheiten (13a, 13b), dass jede Kameraeinheit einen fest vorbestimmten Teil-Aufnahmebereich eines Aufnahmebereichs des Objekts abbildet, und einer mit den Kameraeinheiten verbundenen Bildsyntheseeinheit zur Synthese eines Gesamtbildes des Objekts im Aufnahmebereich aus von den feststehenden Kameraeinheiten erzeugten Teilbildern.

2. Abbildungsanordnung nach Anspruch 1, wobei jede Kameraeinheit einen Bildsensor und eine eigene Abbildungsoptik aufweist.

3. Abbildungsanordnung nach Anspruch 1 oder 2, wobei die Kameraeinheiten derart ausgeführt und angeordnet sind, dass sich die Teil-Aufnahmebereiche mindestens von direkt benachbarten Kameraeinheiten, optional auch der übernächst benachbarten oder noch fernerer Kameraeinheiten, überlappen und die Bildsyntheseeinheit zur Verarbeitung der Überlappungsbereiche ausgebildet ist.

4. Abbildungsanordnung nach einem der vorangehenden Ansprüche, wobei verschiedenartige Kameraeinheiten, insbesondere Farb-Kameraeinheiten und/oder Schwarz-Weiß-Kameraeinheiten und/oder Infrarot-Kameraeinheiten in Zeilen oder Spalten-Gruppen des jeweils gleichen Kameratyps, vorgesehen sind.

5. Abbildungsanordnung nach einem der vorangehenden Ansprüche, wobei der Bildsyntheseeinheit eine Teilbild-Auswahlsteuerung zur Auswahl bestimmter Teilbilder von Kameraeinheiten zur Gewinnung verschiedener Gesamtbilder aus den Teilbildern von Kameraeinheiten eines bestimmten Typs und/oder zur Gewinnung verschiedener Bereichsbilder aus jeweils mehreren den gleichen Objektbereich darstellenden Teilbildern von Kameraeinheiten mit überlappendem Aufnahmebereich zugeordnet ist.

6. Abbildungsanordnung nach einem der vorangehenden Ansprüche, wobei der Bildsyntheseeinheit eine Bildauswertungs-/Vermessungseinheit oder eine Schnittstelle zum externen Anschluss einer solchen zugeordnet ist.

7. Abbildungsanordnung nach einem der vorangehenden Ansprüche, wobei die Kameraeinheiten Bildspeichereinheiten aufweisen und die Bildsyntheseeinheit zum, insbesondere sequentiellen, Auslesen der Bildspeicher ausgebildet ist.

8. Abbildungsanordnung nach einem der vorangehenden Ansprüche, wobei die Beleuchtungseinrichtung eine Vielzahl von in Abstandsbereichen zwischen den Kameraeinheiten angeordneten Leuchtelementen, insbesondere weißen und/oder farbigen LEDs und/oder IR-LEDs, aufweist.

9. Abbildungsanordnung nach einem der vorangehenden Ansprüche, wobei die Beleuchtungseinrichtung hinsichtlich der Beleuchtungs-Lichtfarbe einstellbar ausgeführt ist, insbesondere mehrere wahlweise aktivierbare Gruppen von Leuchtelementen umfasst, die untereinander gleiche, aber zwischen den Gruppen unterschiedliche Lichtfarbe haben.

10. Abbildungsanordnung nach Anspruch 9, wobei eine eingangsseitig mit der Bildsyntheseeinheit und ausgangsseitig mit der Beleuchtungseinheit verbundene Qualitätsprüf-/Steuereinheit vorgesehen ist, die zur selbsttätigen Ansteuerung einer Folge von Abbildungsvorgängen bei unterschiedlicher Lichtfarbe mit jeweils zugeordneter Bildsynthese, zur Auswertung der jeweils aufgenommenen Bilder hinsichtlich der Bildqualität und zur Auswahl einer diesbezüglich optimalen Lichtfarbe der Beleuchtungseinheit ausgebildet ist.

11. Abbildungsanordnung nach einem der vorangehenden Ansprüche, wobei der Beleuchtungseinrichtung Mittel zur Projektion eines vorbestimmten Lichtmusters, insbesondere eines gitterförmigen, streifenförmigen oder punktförmigen Musters, auf die Oberfläche des Objekts und der Bildsyntheseeinheit eine Kalibrierungsstufe zur Kalibrierung des erzeugten Gesamtbildes anhand der in den Teilbildern enthaltenen Lichtmuster-Teile zugeordnet sind.

12. Abbildungsanordnung nach Anspruch 11, wobei auf die Auflageplatte ein Primärmuster zur Bildung des Lichtmusters aufgebracht, insbesondere aufgedruckt oder als separate Musterplatte aufgelegt, ist.

13. Abbildungsanordnung nach Anspruch 12, wobei die Musterplatte ein druckverform bares Muster, beispielsweise eine Matrixanordnung elastisch verformbarer Pyramiden, zur ortsaufgelösten optischen Erfassung des durch das Objekt auf die Auflageplatte ausgeübten Drucks aufweist.

14. Abbildungsanordnung nach einem der vorangehenden Ansprüche, mit einem kastenförmigen Gehäuse, das eine die Auflageplatte haltende und zugleich eine Kamerakammer unterhalb der Auflageplatte und eine Objektkammer oberhalb der Auflageplatte begrenzende, die Kamerakammer und Objektkammer gegenüber Fremdlicht abschirmende Seitenwand hat.

15. Abbildungsanordnung nach einem der vorangehenden Ansprüche, wobei die Kameraeinheiten auf einer gemeinsamen Kameraträgerplatte angebracht sind, welcher mindestens ein Aktor zur Bewegung der Kameraträgerplatte und somit zur gemeinsamen und gleichzeitigen Bewegung aller auf dieser angebrachten Kameraeinheiten in mindestens einer Raumrichtung zugeordnet ist.

16. Abbildungsanordnung nach Anspruch 15, wobei dem oder jedem Aktor eine Kamerabewegungs-Steuereinheit zugeordnet ist, welche über eine Synchronisierungseinheit mit einem Eingang der Bildsyntheseeinheit verbunden ist, und wobei die Kamerabewegungs-Steuereinheit und Bildsyntheseeinheit derart ausgebildet sind, dass die Gewinnung des Gesamtbildes des Objekts in Abhängigkeit von der Bewegung bzw. den Positionen der Kameraträgerplatte während einer Bewegung erfolgt.

17. Abbildungsanordnung nach einem der vorangehenden Ansprüche, wobei der Auflageplatte Druckerfassungsmittel zur, insbesondere ortsaufgelösten, Erfassung des durch das Objekt auf die Auflageplatte ausgeübten Drucks zugeordnet sind.

18. Abbildungsanordnung nach Anspruch 13 und Anspruch 17, wobei die Druckerfassungsmittel durch die Musterplatte mit druckverformbarem Muster und eine zur Auswertung von Verformungen des Musters ausgebildete Muster- Auswertungseinheit gebildet sind, die der Bildsyntheseeinheit nachgeordnet ist.

19. Abbildungsanordnung nach Anspruch 17, wobei die Druckerfassungsmittel eine auf die Auflageplatte aufgebrachte, mindestens im überwiegenden Flächenanteil der Auflageplatte durchsichtige Drucksensormatrix, insbesondere mit kapazitiven Drucksensoren, aufweisen.

20. Abbildungsanordnung nach einem der Ansprüche 17 bis 19, wobei unter der Auflageplatte einzelne Drucksensoren angeordnet sind, welche die Auflageplatte mindestens teilweise abstützen.

21. Abbildungsanordnung nach einem der Ansprüche 17 bis 20, mit einer Drucksignal-Auswertungseinheit oder einer Schnittstelle zum externen Anschluss einer solchen, die insbesondere Mittel zur Synchronisierung mit der Bildsyntheseeinheit zur synchronisierten Gewinnung eines optischen Gesamtbildes und Druckverteilungs-Bildes eines Objekts aufweist.

22. Laufbandanordnung zur Überwachung und/oder zum Training des Gehens oder Laufens, mit einer Abbildungsanordnung nach einem der vorangehenden Ansprüche und einem durchsichtigen Laufband (29c) wobei die Abbildungsanordnung unterhalb einer Lauffläche des Laufbandes angeordnet ist und das durchsichtige Laufband über die Auflageplatte der Abbildungsanordnung bewegt wird oder selbst eine bewegliche Auflageplatte bildet.

## Claims

1. Imaging arrangement (10) for true-angle and depth-focus imaging and optional 3D measurement of spatial objects, with
a transparent support plate (11) for an object to be imaged, which is placed on the support plate,
an illumination device (15) aligned with the support plate to illuminate the object,
a plurality of camera units (13a, 13b) arranged in a matrix substantially fixed in a common plane parallel to the support plate at a predetermined equal distance from the support plate below the support plate in such a way that each camera unit images a fixed predetermined partial recording area of a recording area of the object, and
an image synthesis unit connected to the camera units for synthesising an overall image of the object in the recording area from partial images generated by the fixed camera units.

2. Imaging arrangement according to claim 1, wherein each camera unit comprises an image sensor and its own imaging optics.

3. Imaging arrangement according to claim 1 or 2, wherein the camera units are designed and arranged in such a way that the partial recording areas of at least directly adjacent camera units, optionally also of the camera units that are the next but one or even further away, overlap, and the image synthesis unit is designed to process the overlapping areas.

4. Imaging arrangement according to one of the preceding claims, wherein different types of camera units, in particular colour camera units and/or black-and-white camera units and/or infrared camera units, are provided in rows or column groups of the respective same camera type.

5. Imaging arrangement according to one of the preceding claims, wherein with the image synthesis unit a partial image selection control is associated for selecting specific partial images of camera units for obtaining different overall images from the partial images of camera units of a specific type and/or for obtaining different area images from a respective plurality of partial images of camera units regarding the same object area and having an overlapping recording area.

6. Imaging arrangement according to any of the preceding claims, wherein an image evaluation/survey unit or an interface for the external connection of such is associated with the image synthesis unit.

7. Imaging arrangement according to one of the preceding claims, wherein the camera units have image memory units and the image synthesis unit is designed for reading out, in particular sequentially, the image memories.

8. Imaging arrangement according to one of the preceding claims, wherein the illumination device has a plurality of lighting elements, in particular white and/or coloured LEDs and/or IR LEDs, arranged in spacing regions between the camera units.

9. Imaging arrangement according to one of the preceding claims, wherein the illumination device is designed to be adjustable with regard to the illumination light colour, in particular comprises several selectively activatable groups of lighting elements which have the same light colour among themselves but different light colours between the groups.

10. Imaging arrangement according to claim 9, wherein a quality testing/control unit is provided which is connected on the input side to the image synthesis unit and on the output side to the illumination device and which is designed for the automatic control of a sequence of imaging processes at different light colours with respectively associated image synthesis, for the evaluation of the respectively recorded images with respect to the image quality and for the selection of a light colour of the illumination unit which is optimal in this respect.

11. Imaging arrangement according to one of the preceding claims, wherein with the illumination device means for projecting a predetermined light pattern, in particular a grid-shaped, strip-shaped or point-shaped pattern, onto the surface of the object is associated, and a calibration stage for calibrating the generated overall image on the basis of the light pattern parts contained in the partial images is associated with the image synthesis unit.

12. Imaging arrangement according to claim 11, wherein a primary pattern for forming the light pattern is applied to the support plate, in particular printed on or applied as a separate pattern plate.

13. Imaging arrangement according to claim 12, wherein the pattern plate has a pressure-deformable pattern, for example a matrix arrangement of elastically deformable pyramids, for spatially resolved optical detection of the pressure exerted by the object on the support plate.

14. Imaging arrangement according to any one of the preceding claims, comprising a box-shaped housing having a side wall holding the support plate and simultaneously defining a camera chamber below the support plate and an object chamber above the support plate and for shielding the camera chamber and object chamber from extraneous light.

15. Imaging arrangement according to any one of the preceding claims, wherein the camera units are mounted on a common camera support plate which is associated with at least one actuator for moving the camera support plate and thus for the common and simultaneous movement of all camera units mounted thereon in at least one spatial direction.

16. Imaging arrangement according to claim 15, wherein the or each actuator is associated with a camera movement control unit which is connected via a synchronisation unit to an input of the image synthesis unit, and wherein the camera movement control unit and image synthesis unit are configured such that the acquisition of the overall image of the object takes place in dependence on the movement or positions of the camera support plate during a movement.

17. Imaging arrangement according to one of the preceding claims, wherein pressure detection means for detecting, in particular spatially resolved, the pressure exerted by the object on the support plate are associated with the support plate.

18. Imaging arrangement according to claim 13 and claim 17, wherein the pressure detection means are formed by the pattern plate with pressure-deformable pattern, and a pattern evaluation unit which is designed for evaluating deformations of the pattern and which is arranged downstream of the image synthesis unit.

19. Imaging arrangement according to claim 17, wherein the pressure detection means comprise a pressure sensor matrix, in particular with capacitive pressure sensors, which is applied to the support plate and is transparent at least in the predominant surface portion of the support plate.

20. Imaging arrangement according to any one of claims 17 to 19, wherein individual pressure sensors are arranged under the support plate, which at least partially support the support plate.

21. Imaging arrangement according to any one of claims 17 to 20, comprising a pressure signal evaluation unit or an interface for the external connection of such, which in particular comprises means for synchronisation with the image synthesis unit for synchronised acquisition of an overall optical image and pressure distribution image of an object.

22. Treadmill assembly for monitoring and/or training walking or running, comprising an imaging arrangement according to any one of the preceding claims and a transparent treadmill belt (29c), wherein the imaging arrangement is arranged below a running surface of the treadmill belt and the transparent treadmill belt is moved over the support plate of the imaging arrangement or itself forms a movable support plate.

## Revendications

1. Dispositif d'imagerie (10) pour l'imagerie angulaire et en profondeur de champ et la mesure 3D optionnelle d'objets spatiaux, avec
d'une plaque d'appui transparente (11) pour un objet à reproduire, placé sur la plaque d'appui,
un dispositif d'éclairage (15) orienté vers la plaque d'appui pour éclairer l'objet,
d'une pluralité d'unités de caméra (13a, 13b) disposées en forme de matrice essentiellement de manière fixe dans un plan commun parallèle à la plaque d'appui à une distance égale prédéterminée par rapport à la plaque d'appui en dessous de la plaque d'appui, de telle sorte que chaque unité de caméra reproduit une zone de prise de vue partielle prédéterminée de manière fixe d'une zone de prise de vue de l'objet, et
une unité de synthèse d'image reliée aux unités de caméra pour synthétiser une image globale de l'objet dans la zone de prise de vue à partir d'images partielles générées par les unités de caméra fixes.

2. Dispositif d'imagerie selon la revendication 1, dans lequel chaque unité de caméra comprend un capteur d'image et une optique d'imagerie propre.

3. Dispositif d'imagerie selon la revendication 1 ou 2, dans lequel les unités de caméra sont réalisées et disposées de telle sorte que les zones de prise de vue partielle se chevauchent au moins d'unités de caméra directement voisines, éventuellement aussi des unités de caméra voisines les unes des autres ou encore plus éloignées, et l'unité de synthèse d'images est conçue pour traiter les zones de chevauchement.

4. Dispositif d'imagerie selon l'une des revendications précédentes, dans lequel des unités de caméra de types différents, en particulier des unités de caméra couleur et/ou des unités de caméra noir et blanc et/ou des unités de caméra infrarouge sont prévues dans des lignes ou des groupes de colonnes du même type de caméra.

5. Dispositif d'imagerie selon l'une des revendications précédentes, dans lequel une commande de sélection d'images partielles est associée à l'unité de synthèse d'images pour sélectionner des images partielles déterminées d'unités de caméra afin d'obtenir différentes images globales à partir des images partielles d'unités de caméra d'un type déterminé et/ou pour obtenir différentes images de zones à partir de respectivement plusieurs images partielles représentant la même zone d'objet d'unités de caméra avec une zone de prise de vue qui se chevauche.

6. Dispositif d'imagerie selon l'une quelconque des revendications précédentes, dans lequel l'unité de synthèse d'image est associée à une unité d'évaluation/de mesure d'image ou à une interface de connexion externe d'une telle unité.

7. Dispositif d'imagerie selon l'une des revendications précédentes, dans lequel les unités de caméra comportent des unités de mémoire d'image et l'unité de synthèse d'image est configurée pour lire, notamment de manière séquentielle, les mémoires d'image.

8. Dispositif d'imagerie selon l'une des revendications précédentes, dans lequel le dispositif d'éclairage comprend une pluralité d'éléments lumineux, notamment des LED blanches et/ou de couleur et/ou des LED IR, disposés dans des zones d'espacement entre les unités de caméra.

9. Dispositif d'imagerie selon l'une des revendications précédentes, dans lequel le dispositif d'éclairage est réalisé de manière réglable en ce qui concerne la couleur de la lumière d'éclairage, et comprend en particulier plusieurs groupes d'éléments lumineux pouvant être activés au choix, qui ont la même couleur de lumière entre eux, mais une couleur de lumière différente entre les groupes.

10. Dispositif d'imagerie selon la revendication 9, dans lequel il est prévu une unité de contrôle/commande de la qualité, qui est reliée côté entrée à l'unité de synthèse d'images et côté sortie à l'unité d'éclairage et qui est conçue pour la commande automatique d'une suite de processus d'imagerie pour différentes couleurs de lumière avec une synthèse d'images respectivement associée, pour l'évaluation des images respectivement enregistrées en ce qui concerne la qualité de l'image et pour la sélection d'une couleur de lumière optimale à cet égard de l'unité d'éclairage.

11. Dispositif d'imagerie selon l'une des revendications précédentes, dans lequel des moyens de projection d'un motif lumineux prédéterminé, notamment un motif en forme de grille, de bande ou de point, sur la surface de l'objet sont associés au dispositif d'éclairage et un étage de calibrage est associé à l'unité de synthèse d'images pour calibrer l'image globale produite à l'aide des parties du motif lumineux contenues dans les images partielles.

12. Dispositif d'imagerie selon la revendication 11, dans lequel un motif primaire destiné à former le motif lumineux est appliqué sur la plaque d'appui, en particulier imprimé ou déposé sous forme de plaque à motifs séparée.

13. Dispositif d'imagerie selon la revendication 12, dans lequel la plaque à motifs comprend un motif déformable par pression, par exemple un agencement matriciel de pyramides élastiquement déformables, pour la détection optique à résolution spatiale de la pression exercée par l'objet sur la plaque d'appui.

14. Dispositif d'imagerie selon l'une quelconque des revendications précédentes, comprenant un boîtier en forme de boîte qui présente une paroi latérale maintenant la plaque d'appui et délimitant en même temps une chambre de caméra en dessous de la plaque d'appui et une chambre d'objet au-dessus de la plaque d'appui, et protégeant la chambre de caméra et la chambre d'objet de la lumière extérieure.

15. Dispositif d'imagerie selon l'une quelconque des revendications précédentes, dans lequel les unités de caméra sont montées sur une plaque de support de caméra commune à laquelle est associé au moins un actionneur pour le déplacement de la plaque de support de caméra et donc pour le déplacement commun et simultané de toutes les unités de caméra montées sur celle-ci dans au moins une direction spatiale.

16. Dispositif d'imagerie selon la revendication 15, dans lequel une unité de commande de mouvement de caméra est associée au ou à chaque actionneur, laquelle est reliée à une entrée de l'unité de synthèse d'image par l'intermédiaire d'une unité de synchronisation, et dans lequel l'unité de commande de mouvement de caméra et l'unité de synthèse d'image sont conçues de telle sorte que l'obtention de l'image globale de l'objet s'effectue en fonction du mouvement ou des positions de la plaque de support de caméra pendant un mouvement.

17. Dispositif d'imagerie selon l'une des revendications précédentes, dans lequel des moyens de détection de pression sont associés à la plaque d'appui pour détecter, notamment avec une résolution spatiale, la pression exercée par l'objet sur la plaque d'appui.

18. Dispositif d'imagerie selon la revendication 13 et la revendication 17, dans lequel les moyens d'imagerie sont constitués d'une unité de traitement de l'image.
Les moyens de détection de pression sont constitués par la plaque à motif déformable par pression et par une unité d'évaluation de motif conçue pour évaluer les déformations du motif et placée en aval de l'unité de synthèse d'image.

19. Dispositif d'imagerie selon la revendication 17, dans lequel les moyens de détection de pression comportent une matrice de capteurs de pression transparente, notamment avec des capteurs de pression capacitifs, appliquée sur la plaque d'appui, au moins dans la partie de surface prépondérante de la plaque d'appui.

20. Dispositif d'imagerie selon l'une quelconque des revendications 17 à 19, dans lequel des capteurs de pression individuels sont disposés sous la plaque d'appui et supportent au moins partiellement la plaque d'appui.

21. Dispositif d'imagerie selon l'une quelconque des revendications 17 à 20, comprenant une unité d'évaluation de signal de pression ou une interface de connexion externe d'une telle unité, qui comprend notamment des moyens de synchronisation avec l'unité de synthèse d'image pour l'obtention synchronisée d'une image optique globale et d'une image de répartition de pression d'un objet.

22. Ensemble de tapis roulant pour la surveillance et/ou l'entraînement à la marche ou à la course, comprenant un dispositif d'imagerie selon l'une quelconque des revendications précédentes et un tapis roulant transparent (29c), dans lequel le dispistif d'imagerie est disposé sous une surface de course du tapis roulant et le tapis roulant transparent est déplacé au-dessus de la plaque d'appui de le dispositif d'imagerie ou forme lui-même une plaque d'appui mobile.
